# EUROPEAN PATENT APPLICATION

(11) **EP 1 834 647 A1**
(43) Date of publication of application: **19.09.2007**
(21) Application number: 07003134.9
(22) Date of filing: 14.02.2007
(51) Int. Cl.: A61K 38/17, A23L 1/305

(54) **Canine and equine collagen joint health supplement**

(30) Priority: 14.03.2006 US 782130 P
(71) Applicant: NeoCell Corporation, Inc., Lake Forest, CA 92630 (US)
(72) Inventor: Alkayali, Ahmad, Lake Forest, CA 92630 (US); Quadri, Sarah, Orange, CA 92863 (US)
(74) Representative: Winter, Brandl, Fürniss, Hübner Röss, Kaiser, Polte Partnerschaft Patent- und Rechtsanwaltskanzlei

(57) **Abstract**

A food supplement for administration to mammals, and particularly for dogs and horses, has been shown to have a beneficial effect against degenerative joint conditions. The food supplement includes collagen kolla2®.

## Description

### Cross Reference to Related Applications

This application claims the benefit of Provisional Application No. 60/782,130, filed 14 March 2006, and is a Continuation-in-Part of USSN 11/517,233, filed on 7 September 2006, which is a CIP of USSN 10/909,204, filed on 30 July 2004, which is a CIP of USSN 09/768,141, filed on January 24, 2001, now US patent No. 6,838,440, granted 4 January 2005, and the disclosures of which are incorporated herein by reference to the extent necessary for a full and complete enabling disclosure of this present invention.

### BACKGROUND OF THE INVENTION

### FIELD OF THE INVENTION

The present invention relates generally to a food supplement product and method of use which include a beneficial formulation of ingredients in combination, and which are believed and have been found to have a preventative and remedial effect on connective tissue disorder in mammals. Particularly, the formulations for the food supplements here disclosed are believed and have been found to have a preventative or remedial benefit against joint deterioration in mammals, and particularly in dogs and horses.

Moreover, the present invention provides a composition useful as a dietary food supplement for treating arthritis by oral consumption by mammals. Said composition comprises kolla2® type collagen extracted from desiccated avian sternal cartilage.

The product and method according to the present invention are particularly advantageous for dogs and horses, although the invention is not so limited. That is, the formulations according to the present invention may have beneficial effects for other mammals as well.

### RELATED TECHNOLOGY

Canine Hip Dysplasia (CHD) is a skeletal developmental defect in dogs. A high incidence occurs in larger, rapidly growing dogs or larger working/sporting breeds. The disorder is the result of the upper portion (ball) of the hind legs not fitting properly into the socket of the hip. Dogs are not born with CHD, but as they grow, laxity of muscles and ligaments around the joint in combination with a poor fitness condition produces excess movement at the hip joint. The instability within the joint itself allows the hipbone to pull apart in growth (subluxation).

A general misconception is that CHD is a form of arthritis that affects the hips. Rather severe osteoarthritis is a secondary result of hip dysplasia. It occurs when the bones rub together resulting in irregular bone growth and wear causing osteoarthritis.

It is believed by some, although no substantial proof is given, that CHD is inherited. And many feel the condition is not influenced by diet or caloric intake, but rather is a factor of the animal's overall weight and rapid growth.

Although it cannot be detected at birth, severe cases of CHD may be detectable at six months. The average age for the first symptoms to be displayed is around two years old. Outward signs range from slight to severe pain including difficulty getting up from lying or seated position, climbing stairs, extending back legs, a side to side sway of the crop, a resistance to jumping, a waddling or sway in the gait, lameness especially following exercise, and pushing on the rump may cause the pelvis to drop. Commonly these symptoms are more pronounced on cold, damp days. These are all a result of deterioration of the joint that limits a dog's mobility. CHD may be an inherited trait that is influenced by several genes (polygenic). A licensed veterinarian can properly diagnose CHD through experienced analysis of x-rays of the suspected joint.

Neither the environment nor how a puppy is raised can cause hip dysplasia, however, it could play a role when and perhaps if he/she develops outward symptoms. Some factors that could worsen symptoms of CHD are rough play, jumping, climbing, excess weight gain/rapid growth, calcium supplementation (which increases novel remodeling) or forced distance running especially on tarmac, asphalt or hard surfaces.

There are treatments available for CHD, and some treatments are non-surgical. However, in many cases, only surgery would help to improve function and reduce pain and inflammation. The administration of drugs such as aspirin, phenylbutazone ("bute") or NSAIDs and steroids could all be administered to quell pain, but numerous side effects result from oral pain relievers.

With horses, several causes of joint problems are identified. Horses have joint problems because humans often ask them to do things they weren't designed to do, some believe. This information is provided by the president-elect of the American Association of Equine Practitioners. After domesticating the horse, man designed competitions for him that put a great deal of additional stress on the animal's joints. For example, Dressage seems like a fairly benign competition as far as placing stress on joints is concerned, but that isn't true. The advanced dressage horse is required to move his center of gravity more to the rear, putting more stress on the hind limbs. Some of the lateral movements, such as the shoulder-in and half-pass, cause high joint stress particularly on the hock. The types of disease and injury that can afflict dressage horses include degenerative joint disease of the hocks, inflammation and degenerative joint disease of the front pasterns, inflammation of the middle knee joint, and degenerative joint disease and inflammation of the fetlock.

Similarly, many English show horses also tend to shift their center of gravity to the rear, thus placing more stress on the hind limbs (especially the hock and pastern joints). The goal with some of these show horses is to travel with high front-end action. This is particularly true of the Tennessee Walking Horse in competition. Horses which load more weight on the rear are going to be prone to hock, rear fetlock, and stifle injuries and disease.

With the jumper, there is great stress on the hind limb joints on take-off and on the entire forelimb suspensory apparatus on landing. In addition, the show jumper is often asked to complete one jump, then make a sharp turn to line up for another. This places severe stress on the hocks. Sometimes the stress placed on the joint ligaments of the jumping horse causes inflammation and lameness.

Western horses also are stressed with competition. There is a lot of torque on the rear joints when a cutting horse drops its hindquarters toward the ground and spins a split second before accelerating to stop the movement of the calf it is seeking to hold away from the herd. Some cutting horses are susceptible to injuries and disease involving the hock and stifle joints. The reining horse is asked to run down an arena at speed, slide to a stop, and spin in a circle, with the rear end anchored in place. This produces a great deal of torque on the hind limbs, especially the hocks.

Roping horses also put heavy pressure on their joints. The calf roping horse is asked to slide to a stop as the loop settles over the calf's neck. The sliding stop and the jerk from the calf hitting the end of the rope puts stress on the hock and pastern joints.

The header's team roping horse is asked to swing sideways, pulling a steer into position for the heeler to rope the hind feet. The header's horse places added stress on his lower forelimbs, especially the left, while turning the steer.

A barrel racing horse speeding through the cloverleaf course places severe stress on the joints of his front and rear limbs. There is often a compounding of problems with barrel racers, because in some cases the horses were retired from the racetrack and bring with them problematic front knees, front fetlocks, and front suspensory apparatus.

Western pleasure horses which travel sedately and slowly around the ring might also be prone to joint disease because of their conformation. To accentuate a chosen way of going, he says, many Western pleasure horses have been bred and selected to have straighter shoulders and more upright pasterns than horses which perform at speed. This type of conformation can set the stage for poor shock absorption and thus joint disease.

Finally, breeding practices also are implicated in some joint problems of horses that perform at speed. Cutting horse breeders, for example, often line breed to make certain that the horse has "cow sense." This, however, has the potential for compounding genetic joint problems when conformation isn't also taken into account.

And problems arise. When joints suffer trauma, enzymes and other agents from the joint lining are released that destroy tissue inside the joint, especially articular cartilage (which covers the joint surface of the bone). The result is traumatic arthritis. "Traumatic arthritis," has been defined as the diverse collection of pathological and clinical states which develop after single or repetitive episodes of trauma. The components of traumatic arthritis may include inflammation of the joint lining such as synovitis (inflammation of the synovial membrane) and capsulitis (inflammation of the fibrous joint capsule); injury to the supporting ligaments of the joint (sprain); and fractures to the bones within the joint.

Traumatic arthritis occurs in three forms: Type 1: Synovitis and capsulitis without disturbance of articular cartilage or disruption of major supporting structures. This includes acute synovitis, capsulitis, and most sprains. Type 2: This is caused by disruptive trauma damaging the articular cartilage or completely rupturing major supporting structures. This includes severe sprains, intra-articular fractures, and meniscal tears (the meniscus is cartilage that lies between the weight-bearing surfaces of the joint). Type 3: Post-traumatic degenerative joint disease occurs when there is residual damage after initial trauma. Type 3 traumatic arthritis can lead to deformity, limited range of motion, or joint instability.

Equine joint disease and associated lamenesses are the most common athletic injuries seen in performance horses today. The pathological effect of trauma on joints is to cause synovitis and capsulitis, which, in turn, creates physical and biochemical damage to the articular cartilage. Clinical signs of joint disease include lameness, swelling, excessive synovial fluid, pain on flexion, and heat. However, these signs alone don't tell you what joint structures are affected or how badly they are damaged. A detailed examination is needed to determine a horse's exact problem, treatment regime, and prognosis for future athletic soundness.

Conventional treatments for Equine Joint Disease include intra-articular medication - the direct administration of a drug into a joint. If done correctly, some have had success with this treatment, and maintain that it is a safe way to treat joint disease.

The drugs used most often for the treatment of non-infectious joint conditions, says Black, include polysulfated glycosaminoglycans (PSGAGs), sodium hyaluronate, and corticosteroids. Glycosaminoglycans--PSGAGs can be administered either intra-articularly or by intramuscular injection. The most widely used PSGAG today is Adequan. This medication is used for intra-articular injections weekly for three to five weeks. If the drug is administered intramuscularly, it is given every three to five days for a minimum of four weeks. The PSGAGs can combat elements within the joint that cause inflammation. Studies have shown that PSGAGs stimulate the production of natural hyaluronic acid.

There are advantages and disadvantages to the PSGAG approach. The advantages include beneficial anti-inflammatory effects and chondroprotection (protecting the ends of the bones). These positive effects make this the drug of choice for Type 2 or 3 traumatic joints with damage to the articular cartilage. Disadvantages include a risk of intra-articular reactions unless one also administers appropriate antibiotics, such as amikacin. The antibiotics would be administered at the same time via intra-articular injection. Another disadvantage of PSGAGs is cost. Maintaining a horse on the recommended therapeutic levels can result in significant cost to the owners. Sodium Hyaluronate (also called hyaluronic acid, or HA) is the most recent class of anti-inflammatory medication to be used in the equine joint. This group of therapeutics, provides significant lubrication to the synovial membrane that is responsible for dissipating more than 50% of the friction within the joint. Sodium hyaluronate counteracts metalloproteinases, prostaglandin E2 (involved in several inflammatory processes including the perception of pain), and free radicals.

A synthetic form of sodium hyaluronate--Legend--has been developed that can be administered intravenously or intra-articularly. When given intravenously, Legend has proven to have positive anti-inflammatory effects on the synovial membrane of the traumatized joint, and it has the advantage of offering the veterinarian a method for treating multiple joints with a single injection. Intravenous sodium hyaluronate is given weekly for three to four injections, usually followed by a decreasing number of injections for maintenance. But, it should be recognized that HA is only effective against mild to moderate synovitis, and consequently corticosteroids are often used in conjunction with HA. Although the drug has been proven safe, with few side effects or injection reactions, there are some side effect and injection risks.

Corticosteroids have also been used, but many thought that injecting a joint with corticosteroids was giving the joint a death sentence. Although it would almost immediately reduce the inflammation, in the long run it would have such a deleterious effect that the horse could wind up a cripple.

The current technology suggests that an effective approach involves combining corticosteroids and hyaluronic acid and administering this combination intra-articularly. The combination is believed to provide maximum anti-inflammatory response as well as providing additional lubrication to decrease friction.

### SUMMARY OF THE INVENTION

In view of the apparent absence of a conventional related technology for the treatment of degenerative joint problems in dogs, and the limited and disadvantageous technology for such treatment for equine uses, it is an object of this invention to provide such a expedient.

Particularly, an object for this invention is to provide a food supplement that can be administered to mammals, including dogs and horses, which is free of side effects, and which has a beneficial result against degenerative joint disease.

Yet another object for this invention is to provide such a food supplement which can be used to prevent or treat canine hip dysplasia (CHD).

To this end, the present invention provides a first food supplement formulation presented in the form of pill, tablet, gel cap, capsule, chewable wafer, or liquid, and including: 1000 mg collagen, 5 mg Manganese, and binders.

Also, the present invention provides a second food supplement formulation also presented in the form of a pill, tablet, gel cap, capsule, chewable wafer, or liquid, and including:
700 mg kolla2® collagen, 200 mg MSM, 45 mg CMO, 5 mg Manganese, and binders.

Still another pill, tablet, gel cap, capsule, chewable wafer, or liquid, form of formulation according to this invention is: 500 mg kolla2® collagen, 200 mg Collagen Types 1 and 3, 200 mg MSM, 50 mg HA, 45 mg CMO, and binders.

Finally, a preventative formulation in pill, tablet, gel cap, capsule, chewable wafer, or liquid, form according to this invention includes: 400 mg kolla2® collagen, 400 mg Collagen types 1 and 3, 50 mg Pomegranate extract, 50 mg HA, 5 mg Manganese, and binders.

These formulations are believed to have beneficial results, when administered as a food supplement in an effective dose over extended time periods, especially for the treatment of canine hip dysplasia (CHD).

Other objects, features, and advantages of the present invention will be apparent to those skilled in the art from a consideration of the following detailed description of a preferred exemplary embodiment thereof taken in conjunction with the associated figures which will first be described briefly.

### Brief Description of the Drawings

Figure 1 is a schematic diagram of the process for preparing and the manufacturing of the desiccated sternal avian cartilage powder of the invention;

Figure 2 is a line drawing of a normal canine hip joint;

Figure 3 is a line drawings of an early/mild-stage displasic canine hip joint;

Figure 3A is a print of a radiograph (i.e., an x-ray) of a young (i.e., less than one year of age) large breed canine with degenerative joint disease of the hip (as diagnosed by a licensed surgical veterinarian), a precursor to hip dysplasia, taken on July 1999; and

Figure 4 is a copy of an x-ray of the same canine seen in Figure 3A, taken substantially 7 years after the x-ray of Figure 3A.

### DETAILED DESCRIPTION OF EXEMPLARY PREFERRED EMBODIMENTS OF THE INVENTION

While the present invention may be embodied in many different forms, disclosed herein are several specific exemplary embodiments which illustrates and explains the principles of the invention. In conjunction with the description of these embodiments, methods of use of the inventive food supplement formulations here presented are described. It should be emphasized that the present invention is not limited to the specific embodiments illustrated.

Collagen is a major component of muscles, tendons, cartilage, ligaments, joints and blood vessels. There are four main types of collagen: I, II, III, and IV. Types I and III are primarily found in skin, tendon and bone. In contrast, collagen type II is found predominately in articulator cartilage. Collagen is an unusual protein, in that the proportion of glycine residues is nearly one-third, which is unusually high. Proline is also present to a much greater extent in collagen that in most other proteins. Moreover, collagen contains two amino acids, 4-hydroxyproline and 5-hydroxylysine, that are found in very few other proteins. The amino acid sequence of collagen is remarkably regular, nearly every third amino acid is glycine. In addition, the sequence of glycine-proline-hydroxyproline recurs frequently. In contrast, globular proteins rarely exhibit regularities in their amino acid sequences. *
* Site: (Stryer, L., Biochemistry, Third Edition, W. H. Freeman and Co., New York, 1988, pp.262).

An important constituent ingredient of the formulations according to this invention is kolla2® - desiccated avian sternal cartilage collagen type II powder, the kolla2® powder having an average molecular weight of between about 10,500 and 65,000 daltons.

In one aspect of this preferred art, the kolla2® is obtained from desiccated young avian sternal cartilage. Preferably, the avian sternal cartilage is collected from 4-8 week old chicks. The kolla2® is partially water-soluble and the composition comprises 20% to 30% by weight mucopolysaccaride (carbohydrate) and 65% to 70% by weight Collagen type II (protein) and the content of 1% to 3% by weight lipids is part of the kolla2® composition.

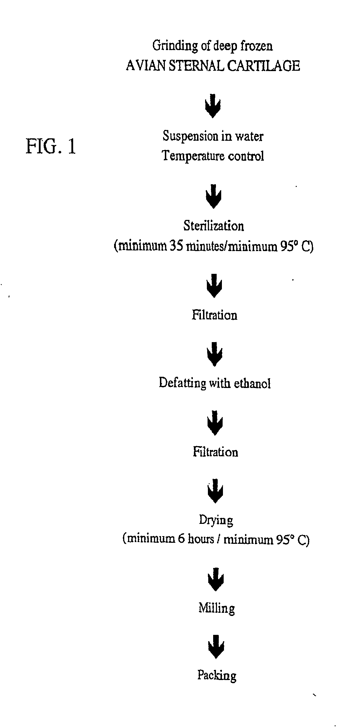

The production of desiccated avian sternal cartilage in powdered form is shown in Figure 1. The method involves cutting fresh sternal cartilage from 4 to 8 weeks avian carcasses and removing all meat, blood and bone therefrom. The sternal cartilage is cut leaving a space of about two and a half millimeters from the bone so as to not remove any bone fragments. This is essential to the purity of the final product because it avoids contamination of collagen type II protein with types I and III, which is found in bone. The fresh sternal cartilage is then promptly deep-frozen. The ground deep-frozen cartilage is suspended in an aqueous solution, preferably water, and sterilized at a controlled temperature at about 95°C for a minimum of 30 minutes. This temperature is high enough to obtain sterility, but not so high that the water boils or reacts with the collagen to hydrolize the collagen. The water is removed by filtration and cartilage mesh is treated with ethanol to remove excess fat, then is filtered and dried at a temperature between about 95°C for a minimum of 6 hours. The dried sternal cartilage is milled to a fine mesh powdered, preferably bulk density at 20°C is approximately about 600 g/l. The powder is partially water-soluble. The average molecular weight of the final kolla2® powder is between 10,500 and 65,000 daltons, and the final powder product is 20% to 30% by weight mucopolysaccharides (particularly chondroitin sulfate and glucosamine sulfate).

Preferred formulations for treatment of degenerative joint problems in canines and equine mammals are set out in Table 1.

**Table 1**

| | |
|---|---|
| Formulation 1: | 1000 mg kolla2® collagen |
| | 5 mg Manganese |
| | binders |
| | |
| Formulation 2: | 700 mg kolla2® collagen |
| | 200 mg MSM (methylsulfonylmethane) |
| | 45 mg CMO (cetylmyristoleate) |
| | 5 mg Manganese |
| | binders |
| | |
| Formulation 3: | 500 mg kolla2® collagen |
| | 200 mg Collagen Type 1 and 3 |
| | 200 mg MSM |
| | 50 mg HA (Hyaluronic acid) |
| | 45 mg CMO |
| | binders |
| | |
| Formulation 4: | 400 mg kolla2® collagen |
| | 400 mg Collagen types 1 and 3 |
| | 50 mg 70% Pomegranate extract |
| | 50 mg HA |
| | 5 mg Manganese |
| | binders |

An actual long-term case study of treatment of canine hip dyspasia has been conducted using the above-identified formulations. In a large breed dog which displayed limping, and obvious pain with reduced mobility, examination showed about 15% loss of hip joint function due to CHD. The animal was diagnosed by a veterinarian as being a candidate for hip replacement. This animal was given a course of treatment using the food supplements according to this invention, with the treatment extending over about 6 years. Subsequent examination showed the animal to be substantially free of hip dysplasia, with improved formation of the head of the hip joint, as well as increased joint spacing, indicating a generation of new cartilage.

When the inventive material, administered in the form of a pill, tablet, gel cap, capsule, chewable wafer, or liquid is taken orally as a daily dietary supplement by an individual with a degenerative joint disorder, the invention helps that individual fabricate cartilage and considerably improves the joint disorder. "Oral" administration includes oral, enteral or intragastric administration. The material of the invention and inventive method can be used to treat, for instance, degenerative joint disease (i.e. rheumatoid arthritis), osteoarthritis, cartilage injuries, joint defects, connective tissue disorder, polychondritis, autoimmune diseases involving connective tissue autoantibodies (i.e. rheumatoid arthritis), and any other connective tissue disorder which would benefit from increased synthesis of cartilage.

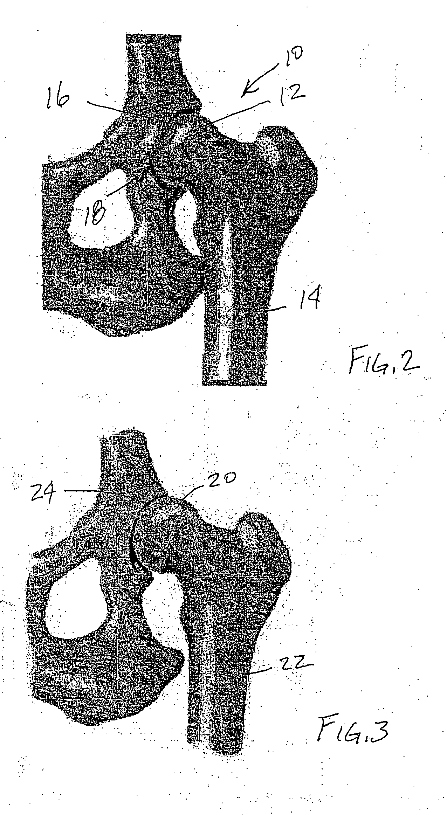

Particularly, as is outlined below, the applicant has discovered and determined that administration orally of the inventive material over an extended period of time is effective in the prevention and/or remediation of degenerative joint disorders in both canines and equines (i.e., dogs and horses). Referring now to Figure 2, it is seen that in the normal canine hip joint 10, the femoral head 12 (i.e., the ball of the hip joint) is disposed at an angle approaching 75 to 80° relative to the shaft 14 of the femur. The acetabular cup 16 is deep, and healthy cartilage, indicated by arrowed numeral 18, is present in the cup and on the ball of the femur. The head of the femur 12 sets tightly within the acetabulum.

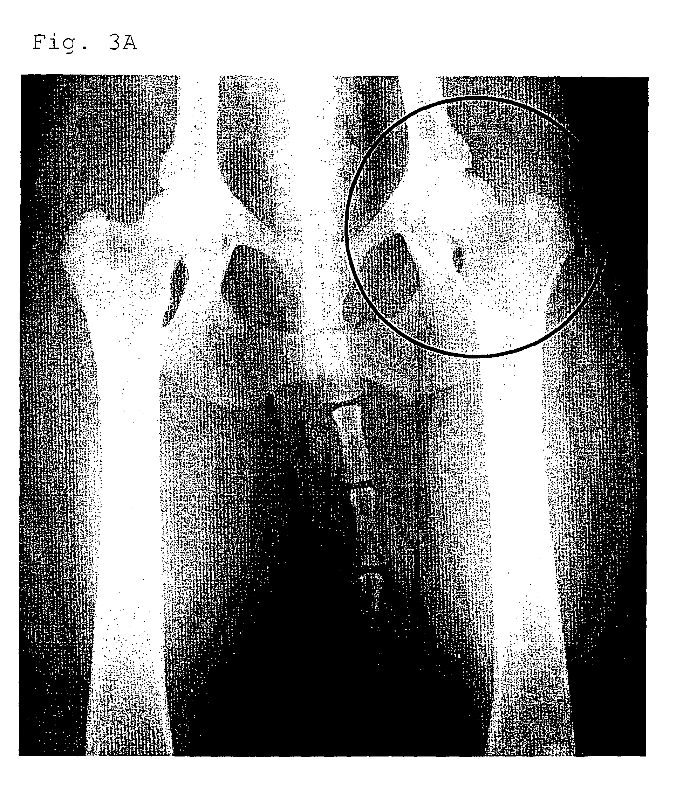

In contrast, viewing Figure 3, a canine case of early/mild displasic hip with no degenerative changes is illustrated. Note how joint laxity allows the head 20 of the femur 22 to subluxate (i.e., move to some extent outwardly of the acetabular cup, rather than being held deeply into the acetabular cup). The acetabular cup 24 may be abnormally shallow and the joint may exhibit laxity. This stage of hip dysplasia will often be treated with corrective surgery before the disease progresses - requiring more invasive and costly surgery.

However, attention now to Figure 3A shows a radiograph of a young large breed canine at an age of less than one year with a hip joint condition substantially as shown in Figure 3. Note particularly in the circled area, illustrating the left hip joint, the apparent condition of joint laxity. The animal was just beginning to show early signs of the hip dysplasia condition. That is, the animal was sporadically but increasingly refusing to bear weight on the hips, or to show normal energetic activity. A veterinary examination (including this x-ray of Figure 3A) led to a diagnosis of bilateral incongruity of the hip joints. Angulation of the femoral heads was noted, and some uncovering of the acetabulum was also noted. A recommendation to begin steroid therapy and to consider surgical correction was given.

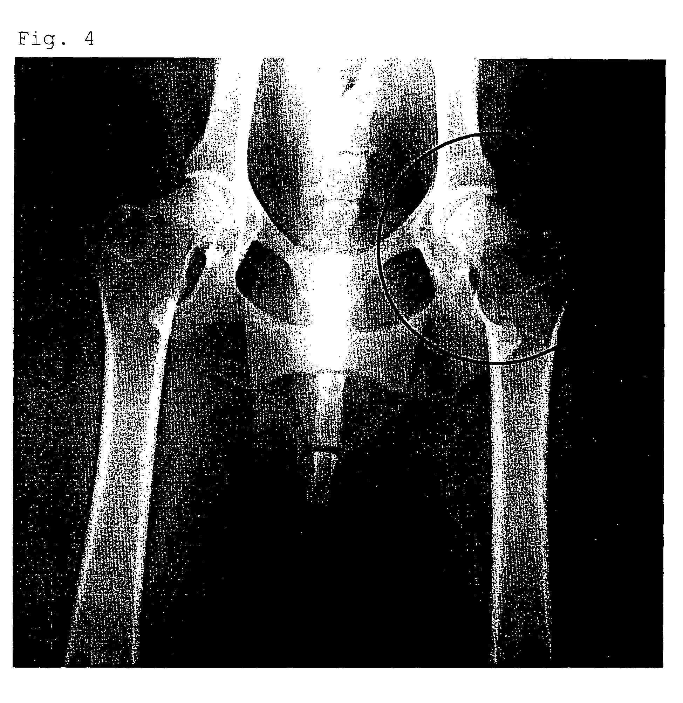

However, Figure 4 shows this same animal after essentially 7 years of supplementation with the present inventive joint health food supplement without use of steroid therapy or surgical correction. As Figure 4 shows, there has been no progression of the hip dysplasia. Note particularly in the circled area of Figure 4, also illustrating the left hip joint, that there is no apparent progression in the condition of joint laxity. In fact, a veterinary examination of this animal conducted in February of 2006 (including the x-ray seen as Figure 4) led to a diagnosis of minimal hip dysplasia, with essentially zero changes in comparison to the condition of the animal in 1999 (recalling Figure 3A).

In addition to the above, the applicant has administered the inventive food supplement to other canines on a trial basis in order to assess the efficacy of the foodsupplement. In one case, the food supplement was assessed by DMV. J. Schreiber, on his patient "Paco." Paco is a small breed dog, an overweight three-legged Chihuahua, with osteoarthritis of the stifle joints, resulting in the dog being a semi-invalid. This animal was born without a front leg. The dog's age at the time of evaluation of the present inventive food supplement was 7 ½ years, and after using the inventive food supplement for an period of 6 months, Dr. Schreiber reported a remarkable difference in the animal's behavior, with the dog being able to move about and even jump. The dog's comfort level was improved at least 75% in the opinion of Dr. Schreiber.

The applicant also participated with the Agoura Animal Shelter of Agoura, California, in an evaluation of the inventive food supplement on several older dogs that were residents at the shelter. When an older dog was noticed to suffer from stiffness of the limbs, they were started on a supplementation program using the present inventive food supplement. In all reported cases, the Agoura Animal Shelter reported that the animals were able to move about with increased comfort, and no signs of stiffness. The Shelter reported that the inventive food supplement allowed the senior dogs to be more comfortable while at the Shelter and helped to make these dogs more viable as adoption candidates.

For oral administration as a nutritional dietary supplement, therapeutic or prophylactic agent, the inventive food supplement may be provided as a dispersible powder or granule, tablet, hard or soft capsule, emulsion, aqueous or oil suspension, syrup or elixir. Compositions intended for oral use may be prepared in accordance with any method known in the art for the manufacturing of nutritional supplement compositions and such compositions may contain one or more of the following components: preservatives, sweeteners, flavoring agents and coloring agents. The flavoring agents and sweetening will enhance the palatability of the preparation.

Tablets containing the inventive material in admixture with non-toxic pharmaceutically acceptable excipients suitable for tablet manufacture are acceptable. Such excipients include inert diluents such as calcium carbonate, sodium carbonate, lactose, calcium phosphate or sodium phosphate, granulating and disintegrating agents, such as corn starch or alginic acid, binding agents such as starch, gelatin or acacia; and lubricating agents such as magnesium stearate, stearic acid or talc. Tablets may be uncoated or may be coated by known techniques to delay disintegration and absorption in the gastrointestinal tract and thereby provide a sustained action over a longer period of time. For example, a time delay material such as glyceryl monostearate or glyceryl distearate alone or with a wax may be employed. The use of enteric coating is also contemplated.

Formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil, liquid paraffin or olive oil.

Aqueous suspensions may contain the powder material of the invention in admixture with excipients suitable for the manufacture of aqueous suspensions. Such excipients include suspending agents, dispersing or wetting agents, one or more preservatives, one or more coloring agents, one or more flavoring agents and one or more sweetening agents such as sucrose or saccharin.

Oil suspensions may be formulated by suspending the active ingredient in a vegetable oil, such as arachis oil, olive oil, sesame oil or coconut oil, or in a mineral oil such as liquid paraffin. The oil suspension may contain a thickening agent, such as beeswax, hard paraffin or cetyl alcohol. Sweeting agents, such as those set forth above, and flavoring agents may be added to provide a palatable oral preparation. These compositions may be preserved by an added antioxidant such as ascorbic acid.

Dispersible powders and granules of the invention suitable for preparation of an aqueous suspension by the addition of water provide the active ingredient in admixture with a dispersing or wetting agent, a suspending agent, and one or more preservatives. Additional excipients, for example, sweetening, flavoring and coloring agents may also be present. Syrups and elixirs may be formulated with sweetening agents, such as glycerol, sorbitol or sucrose. Such formulations may also contain a demulcent, a preservative, a flavoring agent and/or a coloring agent. The inventive material in powder form may be administered in accord with the present inventive method and may be mixed with other ingestible forms and consumed in solid, semisolid solution, suspension or emulsion form. It may also be mixed in conjunction or alternatively with pharmaceutically carriers, flavor enhancers, water, suspending agents and emulsifying agents. In a preferred embodiment, the powder is mixed with a citrus juice such as orange, grapefruit or tangerine due to the promotion of connective tissue formation by ascorbic acid. In a preferred essence, the inventive material may also be formulated in a mixture with ascorbic acid.

For use as a nutritional dietary supplement, prophylactic or therapeutic agent the powder material of this invention according to the method hereof, is orally administered in a daily dosage. For dogs less than 50 pounds, for use as a nutritional dietary supplement, prophylactic or therapeutic agent, kolla2® is orally administered in powder, capsule or tablet form, in a daily dosage of between about 100 mg and 10,000 mg. More preferably, it is administered in a daily dosage of between about 500 mg and 4000 mg. Most preferably, it is administered in a daily dosage 700mg and 1000 mg. For dogs above 50 pounds, for use as a nutritional dietary supplement, prophylactic or therapeutic agent, kolla2® is orally administered in powder, capsule or tablet form, in a daily dosage of between about 500 mg and 10,000 mg. Most preferably, it is administered in a daily dosage between about 700 mg. and 4000 mg. Most preferably, it is administered in a daily dosage between about 1,400 and 3000 mg.

For horses, for use as a nutritional dietary supplement, prophylactic or therapeutic agent, the inventive powder material is also orally administered in a daily dosage generally based on the weight of the animal. For each 100 pounds of animal weight, a preferred dose is from about 100 mg to about 10,000 mg. Alternatively, and more preferably, the daily dose is about 500 mg. to about 3,000 mg. per 100 pounds of animal weight. And still alternatively a most preferred daily dose is from about 1000 mg. to about 2,500 mg.

The preferred embodiment, the supplement is taken on an empty stomach with vitamin C, or the kolla2® powder is mixed with water or a citrus juice prior to ingestion. The preparations described above can be taken indefinitely by dogs or horses affected by connective tissue disorders or by healthy animals as a preventative agent.

The above detailed description of the invention is set forth solely to assist in understanding the invention. It is to be understood that variations of the invention, including all equivalents now known or later developed are to be considered as falling within the scope of the invention, which is limited only by the following claims. Further, those skilled in the art will further appreciate that the present invention may be embodied to the appended claims to define the scope and content of the present invention.

## Claims

1. A method of positively influencing cartilage production and growth in a canine or equine animal with a joint disorder, comprising orally administering to said animal an effective daily cartilage-inducing amount of kolla2®.

2. The method of Claim 1, wherein said connective tissue disorder is selected from the group consisting of degenerative joint diseases, joint defects, hip dysplasia, and osteoarthritis.

3. The method of Claim 1, wherein for a canine less than 50 pounds said effective daily dosage amount is between about 100 mg and 10,000 mg.

4. The method of Claim 1, wherein for a canine less than 50 pounds said effective daily dosage amount is between about 500 mg and 4,000 mg.

5. The method of Claim 1, wherein for a canine less than 50 pounds said effective daily dosage amount is between about 700 mg and 1,000 mg.

6. The method of Claim 1, wherein for a canine weighing more than 50 pounds said effective daily dosage amount is between about 500 mg and 10,000 mg.

7. The method of Claim 1, wherein for a canine weighing more than 50 pounds said effective daily dosage amount is between about 700 mg and 4,000 mg.

8. The method of Claim 1, wherein for a canine weighing more than 50 pounds said effective daily dosage amount is between about 1,400 mg and 3,000 mg.

9. The method of Claim 1, wherein for a horse, for each 100 pounds of body weight, said effective daily dosage amount is between about 100 mg and 10,000 mg.

10. The method of Claim 1, wherein for a horse, for each 100 pounds of body weight, said effective daily dosage amount is between about 500 mg and 3,000 mg.

11. The method of Claim 1, wherein for a horse, for each 100 pounds of body weight, said effective daily dosage amount is between about 1000 mg and 2,500 mg.

12. The method of Claim 1, wherein said effective daily cartilage-inducing amount of kolla2® is administered as a food supplement including in each unit of the food supplement: 1000 mg kolla2® collagen, 5 mg Manganese, and binders.

13. The method of Claim 1, wherein said effective daily cartilage-inducing amount of kolla2® is administered as a food supplement including in each unit of the food supplement: 700 mg kolla2® collagen, 200 mg MSM, 45 mg CMO, 5 mg Manganese, and binders.

14. The method of Claim 1, wherein said effective daily cartilage-inducing amount of kolla2® is administered as a food supplement including in each unit of the food supplement: 500 mg kolla2® collagen, 200 mg collagen Type 1 and 3, 200 mg MSM, 50 mg HA, 45 mg CMO, and binders.

15. The method of Claim 1, wherein said effective daily cartilage-inducing amount of kolla2® is administered as a food supplement including in each unit of the food supplement: 400 mg kolla2® collagen, 400 mg collagen types 1 and 3, 50 mg 70% Pomegranate extract, 50 mg HA, 5 mg Manganese, and binders.

16. A method of daily nutritional supplementation of kolla2® as a preventative for degenerative joint disease in a canine or equine animal, comprising orally administering to an individual animal a daily dosage of kolla2® having an average molecular weight of between about 45,000 and 65,000 daltons, and including the step of administering said kolla2® by selecting a supplementation formula from the group consisting of:
a) in each unit of supplement: 1000 mg kolla2® collagen, 5 mg Manganese, and binders;
b) in each unit of supplement: 700 mg kolla2® collagen, 200 mg MSM, 45 mg CMO, 5 mg Manganese, and binders;
c) in each unit of supplement: 500 mg kolla2® collagen, 200 mg collagen Type 1 and 3, 200 mg MSM, 50 mg HA, 45 mg CMO, and binders; and
d) in each unit of supplement: 400 mg kolla2® collagen, 400 mg collagen types 1 and 3, 50 mg 70% Pomegranate extract, 50 mg HA, 5 mg Manganese, and binders..

17. The method of claim 16 wherein the supplement is in powder form.

18. The method of claim 16 wherein said connective tissue disorder is selected from the group consisting of degenerative joint diseases, joint defects, hip dysplasia, osteoarthritis, polychondritis, and menier's disease.

19. The method of Claim 16, wherein for a canine less than 50 pounds said effective daily dosage amount is between about 100 mg and 10,000 mg.

20. The method of Claim 16, wherein for a canine less than 50 pounds said effective daily dosage amount is between about 500 mg and 4,000 mg.

21. The method of Claim 16, wherein for a canine less than 50 pounds said effective daily dosage amount is between about 700 mg and 1,000 mg.

22. The method of Claim 16, wherein for a canine weighing more than 50 pounds said effective daily dosage amount is between about 500 mg and 10,000 mg.

23. The method of Claim 16, wherein for a canine weighing more than 50 pounds said effective daily dosage amount is between about 700 mg and 4,000 mg.

24. The method of Claim 16, wherein for a canine weighing more than 50 pounds said effective daily dosage amount is between about 1,400 mg and 3,000 mg.

25. The method of Claim 16, wherein for a horse, for each 100 pounds of body weight, said effective daily dosage amount is between about 100 mg and 10,000 mg.

26. The method of Claim 16, wherein for a horse, for each 100 pounds of body weight, said effective daily dosage amount is between about 500 mg and 3,000 mg.

27. The method of Claim 16, wherein for a horse, for each 100 pounds of body weight, said effective daily dosage amount is between about 1000 mg and 2,500 mg.

28. A food supplement for administration to mammals, including: 1000 mg kolla2® collagen, 5 mg Manganese, and binders.

29. A food supplement for administration to mammals, including: 700 mg kolla2® collagen, 200 mg MSM, 45 mg CMO, 5 mg Manganese, and binders.

30. A food supplement for administration to mammals, including: 500 mg kolla2® collagen, 200 mg collagen Type 1 and 3, 200 mg MSM, 50 mg HA, 45 mg CMO, and binders.

31. A food supplement for administration to mammals, including: 400 mg kolla2® collagen, 400 mg collagen types 1 and 3, 50 mg 70% Pomegranate extract, 50 mg HA, 5 mg Manganese, and binders.
